Europäisches Patentamt

European Patent Office

Office européen des brevets

(11) Publication number: **0 238 320**
**A2**

## (12) EUROPEAN PATENT APPLICATION

(21) Application number: **87302314.7**

(22) Date of filing: **18.03.87**

(51) Int. Cl.⁴: **C 12 P 21/00**

(30) Priority: **20.03.86 JP 64411/86**

(43) Date of publication of application:
**23.09.87 Bulletin 87/39**

(84) Designated Contracting States:
**AT BE CH DE ES FR GB GR IT LI LU NL SE**

(71) Applicant: **TAISHO PHARMACEUTICAL CO. LTD**
**24-1 Takata 3-chome Toshima-ku**
**Tokyo 171 (JP)**

(72) Inventor: **Ohno, Tsuneya**
**5-15, Fukasawa 2-chome Setagaya-ku**
**Tokyo (JP)**

**Takeyama, Hiroshi**
**Obayashi Furora 1003, 37-15 Kaminakazato 1-chome**
**Kita-ku Tokyo (JP)**

(74) Representative: **Harrison, David Christopher et al**
**MEWBURN ELLIS & CO 2/3 Cursitor Street**
**London EC4A 1BQ (GB)**

(54) Monoclonal antibody for the antigen specific to Carcinoma of the thyroid.

(57) A monoclonal antibody recognizing the antigen specific to carcinoma of the thyroid can be obtained by homogenizing the cells of human thyroid carcinoma to obtain a finely broken mixture, centrifuging the mixture, centrifuging the supernatant in a discontinuous density gradient sucrose solution, collecting the fraction gathering around the discontinuous density gradient surface, superposing the collected fraction on a continuous density gradient solution of sucrose, subjecting it to ultracentrifuge to separate a fraction having a density of l.l9lp to l.2lOp which is rich in the carcinoma specific antigen, immunizing animals by using the fraction as antigen, preparing a fused cell of the immunized spleen cell and myeloma cell, screening the hybridoma producing the aimed antibody, cloning it, and then culturing it.

The monoclonal antibody specifically exhibits an antigenantibody reactivity only with carcinoma of the thyroid without exhibiting reactivity with benign diseases of the thyroid nor with various carcinomata other than the carcinoma of thyroid.

EP 0 238 320 A2

**Description**

MONOCLONAL ANTIBODY FOR THE ANTIGEN SPECIFIC TO CARCINOMA OF THE THYROID

BACKGROUND OF THE INVENTION

I. FIELD OF THE INVENTION

The present invention relates to a monoclonal antibody specifically reactive with the tissue of human thyroid carcinoma.

2. DESCRIPTION OF THE PRIOR ART

Hitherto, there has been no monoclonal antibody technique applicable to the diagnosis of carcinoma of the thyroid.

As only one monoclonal antibody usable for the diagnosis of adenocarcinoma, that of Japanese Patent Kokai (Laid-Open) No. I8765/85 can be referred to. However, this antibody is specific to adenocarcinoma, and it is unable to differentiate the carcinoma of thyroid from other adenocarcinoma.

Because of its site of occurrence, the carcinoma of thyroid can generally be found out in a relatively early stage. However, it is almost impossible for clinicians to differentiate a carcinoma of thyroid from benign tumors of thyroid.

It can be diagnosed only by studying a pathological tissue specimen prepared from a biopsic material by means of microscope.

Upon the pathological diagnosis, only skillful pathologists (doctors) professional in thyroid can make a right diagnosis. At present, only a few doctors can make such a right diagnosis.

SUMMARY OF THE INVENTION

Under the above-mentioned circumstances, the present inventors conducted earnest studies with the aim of discovering a monoclonal antibody excellently applicable to the diagnosis of carcinoma of thyroid. As the result, there was found a monoclonal antibody having quite excellent properties which exhibits a specific antigen-antibody reactivity only with carcinoma of thyroid, exhibits no reactivity with various benign diseases of thyroid and exhibits no reactivity with various carcinomata other than the carcinoma of thyroid. Based on this finding, the present invention was accomplished.

The present invention provides the following monoclonal antibody and a process for producing said monoclonal antibody:

(I) A monoclonal antibody recognizing the antigen specific to the carcinoma of human thyroid.

(2) A monoclonal antibody of Paragraph I of which molecular weight is I60,000 and of which class-subclass is IgG2a.

(3) A monoclonal antibody of Paragraph I which recognizes the antigen specific to the carcinoma of human thyroid containing a protein component having a molecular weight of about 250,000.

(4) A monoclonal antibody of Paragraph I which can be obtained by homogenizing the cells of human thyroid carcinoma to prepare a finely broken mixture, centrifuging the mixture, taking out the supernatant and centrifuging it in a discontinuous density gradient sucrose solution, collecting the fraction gathering on the discontinuous density gradient surface, superposing it on a continuous density gradient solution of sucrose, subjecting it to ultracentrifuge to separate the fraction having a density of I.I9Ip to I.2I0p which is rich in the carcinoma-specific antigen, immunizing animals by using the fraction as antigen, preparing a fused cell from the immunized spleen cell and myeloma cell, screening out a hybridoma producing the intended antibody, cloning it, and then culturing it.

By the use of the thyroid carcinoma-specific monoclonal antibody of the invention, the above-mentioned problem can be solved. That is to say, by the use of the monoclonal antibody of the invention, only thyroid carcinoma can be stained by a relatively simple procedure so as to differentiate it from other benign tumors and normal thyroid, owing to which even general pathologists (doctors) become able to readily diagnose carcinoma of thyroid and effectively decide the plan for the therapy of this disease.

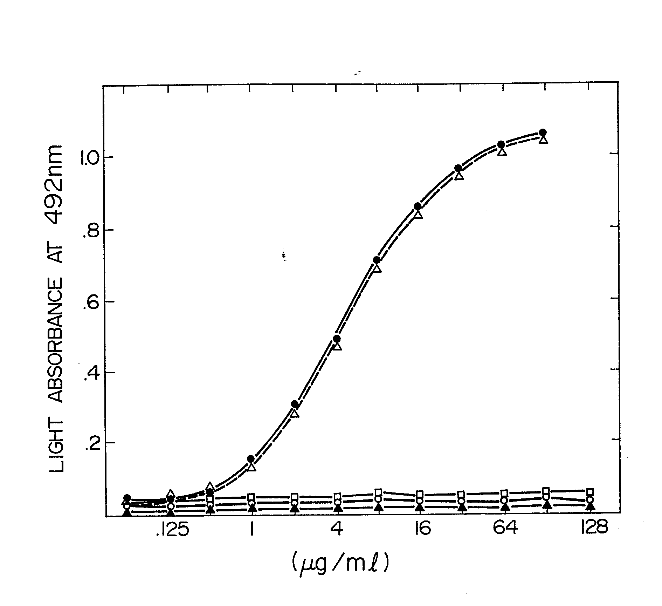

BRIEF DESCRIPTION OF THE DRAWING

The accompanying drawing is a light absorbance distribution diagram illustrating the reaction specificities of the antibody of the invention to various antigens having various concentrations. In the drawing, the symbols express the results of measurement on the following antigens:

0 238 320

the results of measurement on the following antigens:

carcinoma of thyroid        ●————●

carcinoma of thyroid metastatic
to lymph nodes          △-----△

3T3 cell             □————□

T-47D cell           ○————○

KATO III cell          ▲————▲

DETAILED DESCRIPTION OF THE INVENTION
Next, the invention will be concretely explained below.

I. Preparation of Immunogen
A human thyroid carcinoma tissue is cut into small pieces while cooling it with ice, after which the pieces are homogenized while adding slowly thereto an appropriate buffer solution (for example, 5% sucrose-containing Tris-hydrochloric acid buffer solution).

Then, the homogenized mixture is centrifuged for about 10 minutes at about 650 × g to about 1,500 × g to remove the pellets. The supernatant is centrifuged for 10 minutes at about 100,000 × g to precipitate the nucleoli, the mitochondria, etc. Then, the supernatant is superposed on a discontinuous density gradient prepared from a buffer containing 20 to 80% of sucrose (usually, about 25% of sucrose, and buffer containing 45 to 60% of sucrose (usually, about 50% of sucrose), and it is centrifuged for at least about 60 minutes (usually, about 90 minutes) at about 60,000 × g to about 200,000 × g, as the result of which a fraction containing the intended antigen gathers on the discontinuous surface of density gradient. This fraction is taken out and adjusted to a sucrose concentration of about 20% with a buffer. Then, it is superposed on a continuous sucrose density gradient (about 20% to about 50%) using a buffer such as tris-hydrochloric acid buffer and centrifuged at about 60,000 × g to about 200,000 × g for 4 hours or more. The aimed immunogen exists in the fraction having a density of 1.191ρ to 1.210ρ (41.6% to 45.2% in terms of sugar concentration).

This fraction is collected and diluted with tris-hydrochloric acid buffer so that the sugar concentration reaches about 20% or less, and then it is centrifuged at 60,000 × g to 200,000 × g for about 60 minutes or more, usually for about 90 minutes. Thus, the immunogen used in the production according to the present invention is obtained in the form of a pellet.

2. Preparation of Monoclonal Antibody
First, the immunogen of the invention is administered to animals several times together with an appropriate adjuvant (for example, Freund's complete adjuvant) to produce an immune antibody. For example, 1 to 60 micrograns/head of the immunogen of the invention is intraperitoneally or subcutaneously administered to BALB/C mice and, one week or two weeks after it, 1 to 20 micrograms of adjuvant-free immunogen is intravenously administered. By this procedure the animals are immunized.

For preparing antibody-producing cells from immunized animal, the utilization of spleen cells is usually practical. For example, the spleen of the above-mentioned BALB/C mouse is extirpated three days after the administration of the adjuvant-free antigen, and the extirpated spleen is cut into fine pieces in RPMI-1640 or PBS buffer while cooling it. Then it is filtered through stainless steel mesh and adjusted to an appropriate cell number with the above-mentioned buffer.

As the myeloma cell system used for the cell fusion, a myeloma originated from BALB/C mouse MOPC21, such as P3-NSI/1-Ag4-1 (generally called "NS-1") mentioned by Kohler et al. in Eur. J. Immunol. 6, 292-295 (1976), is used, for example. This type of cell system is 8-azaguanine-resistant and lacks hypoxanthinequanine phosphoribosyl transfase, so that it cannot live in HAT medium (hypoxanthine-aminopterin-thymidine-containing medium).

As the cell-fusing agent, polyethylene glycol having an average molecular weight of 1,000 to 6,000 is desirable and Polyethylene Glycol 1500 is particularly preferable. The cell fusing agent is used at a concentration of 40% to 50% depending on its molecular weight. When Polyethylene Glycol 1500 is used, its concentration is adjusted to 50% by the use of RPMI medium 1640. In carrying out the cell fusion, the above-mentioned antibody-producing cell and myeloma cell are washed with RPMI medium 1640 buffer, and the former and the latter are mixed together at a ratio of 4 : 1 to 10 : 1. The mixture is precipitated by centrifugation at 800 × g for 5 minutes and the medium is decanted. To the pellet thus obtained, about one milliliter of the above-mentioned cell fusing agent, per 1 to 2 × 10⁶ cells, is dropped at 37°C over a period of one minute with gentle stirring. Then, with continued stirring, it is slowly suspended into RPMI medium 1640 (37°C, 5 to 10 ml) over a period of 5 to 10 minutes. After centrifuging it at 400 to 600 × g for 5 minutes, the

3

supernatant is decanted and the precipitate is adjusted to a cell number of 1-2 × 10⁶/ml by the use of 15% horse serum-containing RPMI medium 1640. Recommendably, selection of hydridoma is carried out by a culture in HAT medium (HAT selection). First, 0.1 ml each of the cell suspension which has completed the cell fusion is poured into each well of a 96 well type tissue culture plate and cultured at 37°C for 24 hours, after which 0.1 ml each of HAT medium is added thereto. Thereafter, the culture is continued for 3 weeks during which a half quantity of the culture fluid is replaced with HAT medium at intervals of 1 to 4 days. As the result, the parent cells all die and only the fused cells grow up. Thereafter, they are cultured in 15% horse serum-containing RPMI medium 1640 to produce the antibody. Then, whether the aimed antibody has been produced from the hydridoma or not is checked by an appropriate method, such as enzymic immunoassay (ELIS Assay).

3. Screening of Antibody

The screening of the thyroid carcinoma-specific monoclonal antibody is carried out according to the enzymic immunoassay (ELIS Assay) by using the antigens prepared from human thyroid carcinoma tissue, thyroid carcinoma tissue metastatic to lymph nodes and cultured cells of the cell line 3T3 (a cell line originated from normal mouse cell, deposited in ATCC as CCL92), cell line T-47D (a cell line isolated and established from cancer of human breast, deposited in ATCC as HTB-133) and cell line KATO III (a cell line isolated and established from cancer of human stomach, offered by Prof. Watanabe, Niigata University), which are freely available, and have been prepared according to the above-mentioned preparation of immunogen.

First, the antigen is prepared, and then it is diluted with sodium carbonate buffer (pH 9.5) to a concentration of 3 to 5 micrograms/ml and the dilution is coated into each well of 96 well type micro-titer plate. After allowing the plate to stand overnight at 4°C, it is thoroughly washed with phosphate buffer (PBS) containing polyoxyethylene sorbitan monolaurate (Tween 20). Then, after subjecting it to, a blocking process using horse serum, for example, 0.1 ml each of the above-mentioned hybridoma culture solution is added and allowed to stand at 37°C for 5 to 6 hours.

Then, the culture fluid is decanted and the residue is thoroughly washed with the above-mentioned PBS buffer, after which 1% normal goat serum is added and the mixture is allowed to stand for one hour to block non-specific adsorptions. Then, after washing it with the above-mentioned PBS buffer, goat anti-mouse globulin peroxidase conjugated IgG is added and incubated at 37°C for 4 hours. Then, it is thoroughly washed with the above-mentioned PBS buffer.

o-Phenylenediamine dihydrochloride as a substrate and hydrogen peroxide-containing sodium citrate buffer are added, and light absorbance is measured 30 minutes after it. Using a culture fluid of a cell not producing antibody as a control, the difference in absorbance in each well is checked. A sample assuming a significant dyestuff reaction is taken as positive.

As the intended antibody, that positive to the antigens originated from human thyroid carcinoma and the thyroid carcinoma metastatic to lymph nodes and negative to the antigens originated from cell lines 3T3, T-47D and KATO III is selected. Further, after ascertaining the specificity to phathological specimen, a hybridoma producing it is obtained.

4. Method for Staining Pathological Specimen

Pathological specimen is prepared from formalin-fixed paraffin specimen of human carcinoma tissue.

After removing paraffin with xylene, the specimen is stepwise treated with ethanol ranging from 100% ethanol to 70% ethanol, and finally it is thoroughly washed with PBS buffer. Then the specimen is treated with hyaluronidase at 37°C for one hour, washed with PBS buffer and then incubated with trasylol containing normal goat serum at 37°C for 10 minutes. After washing it with PBS buffer, it is reacted with the above-mentioned screened monoclonal antibody at 37°C for 20 minutes. After again washing it with PBS buffer, peroxidase conjugated IgG fraction of goat anti-mouse globulin is added, and it is incubated at room temperature for 30 minutes. Further, after washing it with PBS buffer, it is dipped for 10 minutes in a PBS buffer containing hydrogen peroxide and saturated with DAD (3,3′-diaminobenzidine). Then, the specimen is washed with PBS buffer, stained with methylene blue in the usual way, dehydrated with ethanol and washed with xylene to obtain a pathological specimen. It is examined by means of microscope.

5. Screening of Antibody-producing Cell

The aimed carcinoma-specific antibody-producing hybridoma is selected and cultured by appropriate methods to obtain single clonal cell strain originated from single cell (cloning). Limiting dilution method is usually adopted.

For example, thymus cell or spleen cell of BALB/C mouse is used as feeder, and it is adjusted to a concentration of 1 to 5 × 10⁷/ml. Its 0.1 ml portion is poured into each well of a 96 well type tissue culture plate. Then, a thin suspension of the above-mentioned screened antibodyproducing hybridoma is prepared and dividingly poured into each well of the plate.

The feeder and the hybridoma are suspended in HAT medium or horse or fetal calf serum-containing RPMI-1640 medium, and the suspension is so prepared that one hybridoma enters every one well. Addition of feeder is not always necessary. Then, it is cultured at 37°C in 5% CO₂ culturing apparatus. As the result, clones grow up in 1 to 2 weeks. Under microscope, those wells in which only one clone is growing in one well are selected, and they are analyzed according to the above-mentioned screening method to screen out the clone producing the aimed antibody.

6. Mass Production of Antibody

From the screened hybridoma, antibody can be produced by any of in vitro culture and in vivo culture.

The in vitro culture is carried out by culturing hybridoma in an appropriate nutrient medium, such as RPMI-I640 medium containing fetal calf serum or house serum, for an appropriate period of time. It is suitable for obtaining a pure monoclonal antibody containing no other immunoglobulin. For producing a large amount of monoclonal antibody, in vivo culture is suitable.

It is practised, for example, by the following procedure. Thus, 0.5 ml of 2,6,l0,l4-tetramethylpentadecane (Pristan) is intraperitoneally administered to BALB/C mice. After 2 to 20 days have passed, I to 5 × I0$^6$ cells of screened hybridoma are intraperitoneally given. In 2 to 3 weeks, the grown and fixed hybridoma is a strain which forms ascites tumor. By intraperitoneally administering the strain to necessary heads of mice, ascites containing the aimed monoclonal antibody can be continuously taken out from 2 to 3 weeks after the administration. By verifying its specific activity of antibody according to an appropriate method and purifying it by an appropriate purifying means such as salting-out using ammonium sulfate, ion exchange using DEAE-cellulose or the like, gel filtration, affinity chromatography and the like, a sample having a high purity can be obtained.

7. Characteristic Properties of Monoclonal Antibody

Characteristic properties of the monoclonal antibody exhibiting a specific reactivity with thyroid carcinoma, thus obtained, are as follows:

```
(1)  Molecular weight                   ca. 160,000

(2)  Class-subclass                     IgG2a

(3)  Antigen substance which it         High polymeric
     recongizes                         substance hav-
                                        ing a molecular
                                        weight of ca.
                                        250,000

(4)  Reaction specificity
```

(4-I) Its reaction specificities with antigens prepared from a thyroid carcinoma tissue, a tissue of thyroid carcinoma metastatic to lymph nodes and cultured cells of cell lines 3T3, T-47D and KATO III were studied by the same enzymatic immunoassay (ELIS Assay) as used in the screening of antibody. As the result, as shown in Figure I, it was intensely reactive only with the antigens prepared from the thyroid carcinoma tissue and the tissue of thyroid carcinoma metastatic to lymph nodes and exhibited no reactivity at all with antigens prepared from mouse normal cell 3T3, human breast cancer cell line T-47D and human stomach cancer cell line KATO III.

(4-2) Samples taken from 48 cases of thyroid carcinoma were stained by the enzymatic immunohistochemical technique detailed in the paragraph concerning staining of pathological specimen. As the result, all the 48 samples were positive. On the other hand, no reaction was observable at all in the normal thyroid part of said tissue specimens.

Table 1

|  | No. of stained cases | No. of positive cases | Percentage of positive cases |
|---|---|---|---|
| Thyroid carcinoma tissue | 48 | 48 | 100 |
| Normal thyroid tissue adjacent to carcinoma cell | 48 | 0 | 0 |

(4)-3 Tissues of thyroid adenoma, normal thyroid adjacent to thyroid adenoma, chronic thyroiditis, hyper thyroidismus and adenomatous goiter were stained by the same method as above. As the result, all the cases were negative as shown in Table 2.

Table 2

| Tissue | No. of stained cases | No. of positive cases |
|---|---|---|
| Thyroid adenama | 14 | 0 |
| Normal thyroid adjacent to thyroid adenoma | 14 | 0 |
| Chronic thyroiditis | 2 | 0 |
| Hyper thyroidismus | 1 | 0 |
| Adenomatous goiter | 6 | 0 |

(4)-4 Reactivities with carcinoma tissues other than thyroid carcinoma were investigated by the above-mentioned method. As shown in Table 3, the monoclonal antibody of the invention did not reacted at all with carcinomata of pharynx, esophagus, lung, mammillary, stomach, panchreas, gall bladder, ovary, uterus and testicle and glioma.

## Table 3

| Carcinoma tissue | No. of stained cases | No. of positive cases |
|---|---|---|
| Pharynx | 1 | 0 |
| Esophagus | 2 | 0 |
| Lung | 4 | 0 |
| Mammillary | 6 | 0 |
| Stomach | 4 | 0 |
| Pancreas | 3 | 0 |
| Gall bladder | 2 | 0 |
| Ovary | 1 | 0 |
| Uterus | 3 | 0 |
| Testicle | 2 | 0 |
| Glioma | 1 | 0 |
| Total | 29 | 0 |

As above, the monoclonal antibody of the invention specifically reacts only with carcinoma of the thyroid and does not react with various benign diseases of thyroid nor with carcinomata other than carcinoma of thyroid. Accordingly, it is quite useful for diagnosing carcinoma of thyroid.

Next, the invention will be explained with reference to the following examples.

Example I: Preparation of Monoclonal Antibody

I-(I) Preparation of Immunogen

A human thyroid carcinoma tissue was cut into small pieces while cooling it with ice, and the cut pieces were homogenized with cell breaking apparatus in 5% (W/W) sucrose-containing TNE buffer [0.0IM Tris-hydrochloric acid buffer (pH 7.5), 0.I5M NaCl, 0.003M EDTA (pH 8.0)]. The Tris-hydrochloric acid buffer was added at a proportion of 4 ml per I g of carcinoma tissue, and mashing for I5 seconds followed by cooling for 30 seconds was repeated four times. Then, it was centrifuged at 650 × g for I0 minutes. Thus nucleoli and mitochondria precipitated, and there was obtained a supernatant containing the aimed immunogen component (sup-2).

Then, a discontinuous density gradient consisting of 8 ml of 20% (W/W) sucrose-containing TNE buffer and 6 ml of 50% (W/W) sucrose-containing TNE buffer was prepared, and 25 ml of the above-mentioned supernatant (sup-2) was superposed thereon, after which the whole was centrifuged at I00,000 × g for 90 minutes in sw27 swinging bucket. The fraction containing the aimed immunogen was obtained at the interface between the two buffers having different densities. Then, a continuous density gradient was prepared from I5 ml of 20% (W/W) sucrose-containing TNE buffer and I5 ml of 50% (W/W) sucrose-containing TNE buffer. The above-mentioned sample obtained at the density interface of discontinuous density gradient centrifuge was made into a solution having a sucrose concentration of 20% with TNE buffer, and its I0 ml was superposed on the continuous density gradient and centrifuged at I00,000 × g for I2 hours by means of sw27 swinging bucket. After centrifugation, the sample was withdrawn from the bottom of the swinging bucket in I.2 ml portions to obtain 32 to 33 fractions. The density of sugar was measured, and fractions having a density of I.I9Ip to I.2I0p

7

(41.6% to 45.2% in terms of sugar concentration) were combined. Its sugar concentration was adjusted to 20% or below with TNE buffer, and then it was centrifuged at 100,000 × g for 90 minutes. By collecting the pellet, the aimed immunogen was obtained.

I-(2) Immunization of Mouse Spleen Cell

Fifty micrograms of the immunogen prepared in Example I-(I) was dissolved into 0.3 ml of PBS buffer containing an equivalent quantity of Freund's complete adjuvant and intraperitoneally administered to 6 weeks old BALB/C or NZB mice. One week after, 25 micrograms of the same immunogen as above was dissolved in 0.3 ml of PBS buffer containing an equivalent quantity of Freund's complete adjuvant and intraperitoneally administered. Further, after an additional one week, 5 micrograms of the same immunogen as above was dissolved in 0.3 ml of PBS buffer and administered into the tail vein. Three days after it, the spleen was extirpated, washed with PBS buffer and filtered through stainless steel mesh to obtain a suspension of single cells. Then, its cell number was adjusted to 2 × 10⁶/ml with PBS buffer.

I-(3) Fusion of Immunized Spleen Cell and Mouse Myeloma Cell

The mouse myeloma cell used herein was that derived from BALB/C mouse MOPC2I as mentioned in, for example, Kohler et al.: Eur. J. Immunol. 6, 292-295 (1976), and it was called mouse myeloma NS-I. First, mouse myeloma cells of the logarithmic phase having been cultured in RPMI-I640 complete medium (glutamine 2 mM, sodium pyruvate I mM, Fungizone 0.25 microgram/ml, Streptomycin 50 micrograms, Penicillin 50 units/ml, horse serum 15%) were washed with serum-free RPMI-I640 medium, and the cell number was adjusted to 5 × 10⁵/ml with horse serum-free RPMI I640 medium. Then, 40 ml of the immunized mouse spleen cell suspension prepared in Example I-(2) and 10 ml of the above-mentioned mouse myeloma cell suspension were mixed together and the mixture was centrifuged at 800 × g for 5 minutes to form a pellet. The supernatant was completely removed by decantation. To the pellet, I ml of a polyethylene glycol solution (a solution of PEG-I500 of which concentration had been adjusted to 50% with horse serum-free RPMI I640) warmed to 37°C was slowly added with stirring, and the resulting mixture was stirred for one minute. Then, 6 ml of horse serum-free RPMI I640 was slowly added thereto over a period of 6 to 7 minutes with continued stirring. After centrifuging it at 600 × g for 5 minutes and removing the horse serum-free RPMI medium I640, RPMI medium I640 was added to prepare a suspension having a cell number of 2 × 10⁶/ml.

I-(4) HAT Selection

The cell suspension prepared in Example I-(3) was dividingly poured in 0.I ml portions into each well of 96 well type tissue culture plate and cultured at 37°C in a 5% CO₂ culturing apparatus. Twenty four hours after, 0.I ml of HAT medium [RPMI-I640 containing hypoxanthine, aminopterin and thymidine; Littlefield: Science 145, 709-710 (1964)] was added to the culture fluid of each well, after which 0.I ml of culture fluid was removed from each well and the culture was continued. Further, 2, 3, 5, 8, II, I4, I7 and 2I days after the starting of the culture, a half quantity of culture fluid was replaced with HAT medium in the same manner as above. By this HAT selection, all the myeloma cells died and only fused cells grew up. The grown hybridoma was cultured in horse serum-containing RPMI-I640, and the culture fluid was used as the sample for enzymic immunoassay (ELIS Assay).

I-(5) Screening of Antibody by Enzymic Immunoassay (ELIS Assay)

Enzymic immunoassay (ELIS Assays) were carried out, using the antigens prepared from human thyroid carcinoma tissue, thyroid carcinoma tissue metastatic to lymph nodes and cultured cells of cell line 3T3, cell line T-47D and cell line KATO III according to the method of Example I-(I). These antigens were diluted with sodium carbonate buffer (0.05M NaHCO₃, 0.05M Na₂CO₃, pH 9.5) to a concentration of 4 micrograms/ml. Then, the dilutions were dividingly poured in 0.I ml portions into each well of a 96 well type microtiter plate, and the surface of the plate was sealed, after which it was allowed to stand overnight at 4°C. Next morning, the plate was thoroughly washed with PBS buffer containing 0.05% of polyoxyethylene sorbitan monolaurate (Tween 20), and 0.I ml of the hybridoma culture fluid prepared in Example I-(4) was added to each well. The plate was allowed to stand at 37°C for 6 hours. As control, a culture fluid not producing antibody was used.

The culture fluid was decanted, the residue was five times washed with PBS buffer containing 0.05% of Tween 20, 0.I ml of PBS buffer containing I% of normal goat serum was added to each well, the plate was allowed to stand at 37°C for one hour, and then it was thoroughly washed with PBS buffer containing Tween 20.

Then, 0.I ml of horseradish peroxidase conjugated IgG fraction of goat anti-mouse globulin diluted with PBS buffer to I : 30 was added to each well and incubated at 37°C for 4 hours. Each well was four times washed with PBS buffer containing Tween 20, and I/I00 volume of 40 mM ABTS [diammonium 2,2'-azino-di-(3-ethylbenzo-thiazoline)-sulfonate] and I/I00 volume of 0.5% sodium citrate-phosphate buffer containing 0.005% of hydrogen peroxide (pH 5.8) were added as substrate. Thirty minutes after, light absorbance of each well was measured. By referring to control, samples exhibiting a significant color reaction were taken as positive. According to this precedure, samples reactive with the antigens prepared from thyroid carcinoma tissue and thyroid carcinoma tissue metastatic to lymph nodes and unreactive with the antigens prepared from cell lines 3T3, T-47D and KATO III were screened out. Further, reaction specificity to pathological tissue specimen was verified, and cloning was carried out according to the limiting dilution method.

8

I-(6) Standing of Tissue Specimen

Formalin-fixed paraffin specimens of human thyroid carcinoma tissue and thyroid carcinoma tissue metastatic to lymph nodes were dipped in xylene to remove the paraffin. Then, they were stepwise dipped into 100% to 70% ethanol solutions and finally they were thoroughly washed with PBS buffer.

Then, each specimen was reacted with 0.15 ml of hyaluronidase (600 units/ml 0.01M PBS buffer, pH 6.5) at 37°C for one hour. After washing it with PBS buffer, it was incubated with normal goat serum (adjusted to 50% concentration with PBS buffer) containing 0.5 unit/ml of protease inhibitor (trasylol) at 37°C for 10 minutes to block the non-specific adsorption to tissue. After gently washing it with PBS buffer, it was incubated with the monoclonal antibody screened out in Example I-(5) at 37°C for 20 minutes. After three times washing it with PBS buffer, horseradish peroxidase conjugated IgG fraction of goat anti-mouse globulin (adjusted to 100 micrograms IgG/ml total goat serum with PBS buffer) was allowed to act upon it at room temperature for 30 minutes. After again washing it with PBS buffer, it was dipped in PBS buffer saturated with DAD (3,3′-diaminobenzidine) and containing 0.005% hydrogen peroxide at room temperature for 10 minutes. Then, it was washed with PBS buffer, stained with methylene blue, stepwise dehydrated with 70% to 100% ethanol and finally washed with xylene to obtain a pathological specimen.

I-(7) Limiting Dilution Method

The hybridoma screened out in Example I-(5) was cultured in horse serum-containing RPMI medium 1640 and then diluted with horse serum-containing RPMI medium 1640 so that cell number reaches 100/ml, 50/ml, 10/ml and 5/ml. Then, each suspension was dividingly poured in 0.1 ml portions into 96 well type tissue culture plate and cultured. The culture fluid of the well where cell growth was noticeable was tested for production of antibody, according to the enzymic immunoassay (ELIS assay) mentioned in Example I-(5). This procedure was twice repeated to obtain single clonal cells.

I-(8) Mass Production of Antibody

0.5 ml of Pristan was intraperitoneally administered to BALB/C mice. Twenty days after it, the animals were inoculated with 2 × 10$^6$ cells of the single clonal hybridoma obtained in Example I-(7). Ten to twenty days after it, ascites tumor appeared, from which strains of ascites tumor were obtained. By administering about 2 × 10$^6$ cells of this ascites tumor strain intraperitoneally to other plural BALB/C mice, ascites containing the aimed monoclonal antibody became obtainable from the time 2 to 3 weeks after the administration.

I-(9) Purification of Antibody

To 10 ml of the antibody-containing ascites obtained in Example I-(8), an equivalent quantity of PBS buffer was added and then 20 ml of 36% solution of Na$_2$SO$_4$ was added so as to prepare a 18% Na$_2$SO$_4$ solution ultimately. It was centrifuged at 10,000 r.p.m. for 15 minutes, the supernatant was removed, and the pellet was dialyzed against PBS buffer overnight. Then, it was adsorbed on Protein A Sepharose 4B column, and the fractions eluted with acetate buffer (0.1M acetic acid, 0.14M NaCl, pH 5.0, 4.3) were pooled. An analysis of the product had revealed that the monoclonal antibody thus obtained has a molecular weight of ca. 160,000 and its class-subclass was IgG2a.

**Claims**

1. A monoclonal antibody recognizing the antigen specific to human thyroid carcinoma.

2. A monoclonal antibody according to Claim 1, of which molecular weight is 160,000 and of which class-subclass is IgG2a.

3. A monoclonal antibody according to Claim 1 recognizing human thyroid carcinoma-specific antigen containing a protein component having a molecular weight of ca. 250,000.

4. A monoclonal antibody according to Claim 1 which is obtained by homogenizing human thyroid carcinoma cells to obtain a finely broken mixture, centrifuging the mixture, centrifuging the supernatant in a discontinuous sucrose density gradient solution, collecting the fraction gathering on the discontinuous surface of density gradient, superposing it on a continuous sucrose density gradient solution, subjecting it to ultracentrifuge to separate a fraction having a density of 1.191p to 1.210p which is rich in carcinoma-specific antigen, immunizing an animal by using it as antigen, preparing a fused cell of the immunized spleen cell and myeloma cell, screening out the hybridoma producing the aimed antibody, cloning it, and then culturing it.

5. A process for producing a monoclonal recognizing human thyroid carcinoma-specific antigen which comprises homogenizing human thyroid carcinoma cells to obtain a finely broken mixture, centrifuging the mixture, centrifuging the supernatant in a discontinuous sucrose density gradient solution, collecting the fraction gathering on the discontinuous surface of density gradient, superposing it on a continuous sucrose density gradient solution, subjecting it to ultracentrifuge to separate a fraction having a density of 1.191p to 1.210p which is rich in carcinoma-specific antigen, immunizing an animal by using it as antigen, preparing a fused cell of the immunized spleen cell and myeloma cell, screening out the hybridoma producing the aimed antibody, cloning it, and culturing it.

6. A process for producing a monoclonal antibody according to Claim 5, wherein the monoclonal antibody has a molecular weight of l60,000 and class-subclass of IgG2a.

7. A process for producing a monoclonal antibody according to Claim 5, wherein the monoclonal antibody recognizes a human thyroid carcinoma-specific antigen containing a protein component having a molecular weight of ca. 250,000.

Claims for the following Contracting States: AT, GR, ES.

l. A process for producing a monoclonal recognizing human thyroid carcinoma-specific antigen which comprises homogenizing human thyroid carcinoma cells to obtain a finely broken mixture, centrifuging the mixture, centrifuging the supernatant in a discontinuous sucrose density gradient solution, collecting the fraction gathering on the discontinuous surface of density gradient, superposing it on a continuous sucrose density gradient solution, subjecting it to ultracentrifuge to separate a fraction having a density of l.l9lρ to l.2lOρ which is rich in carcinoma-specific antigen, immunizing an animal by using it as antigen, preparing a fused cell of the immunized spleen cell and myeloma cell, screening out the hybridoma producing the aimed antibody, cloning it, and culturing it.

2. A process for producing a monoclonal antibody according to Claim l, wherein the monoclonal antibody has a molecular weight of l6O,OOO and class-subclass of IgG2a.

3. A process for producing a monoclonal antibody according to Claim 2, wherein the monoclonal antibody recognizes a human thyroid carcinoma-specific antigen containing a protein component having a molecular weight of ca. 25O,OOO.